# EUROPÄISCHE PATENTSCHRIFT

(11) **EP 3 051 926 B1**
(45) Veröffentlichungstag und Bekanntmachung des Hinweises auf die Patenterteilung: **27.10.2021**
(21) Anmeldenummer: 16152759.3
(22) Anmeldetag: 26.01.2016
(51) Int. Cl.: H05H 1/24, A61L 2/14

(54) **PLASMAERZEUGUNGSEINRICHTUNG, PLASMAERZEUGUNGSSYSTEM, VERFAHREN ZUR ERZEUGUNG VON PLASMA UND VERFAHREN ZUR DESINFEKTION VON OBERFLÄCHEN**
PLASMA GENERATING DEVICE, PLASMA GENERATING SYSTEM, METHOD OF GENERATING PLASMA AND METHOD FOR DISINFECTING SURFACES
DISPOSITIF DE PRODUCTION DE PLASMA, SYSTEME DE PRODUCTION DE PLASMA, PROCEDE DE PRODUCTION DE PLASMA ET PROCEDE DE DESINFECTION DE SURFACES

(30) Priorität: 30.01.2015 DE 102015101391
(43) Veröffentlichungstag der Anmeldung: 03.08.2016
(73) Patentinhaber: Leibniz-Institut für Plasmaforschung und Technologie e.V., 17489 Greifswald (DE)
(72) Erfinder: Baudler, Jan-Simon, 17491 Greifswald (DE); Turski, Philipp, 17489 Greifswald (DE); Weltmann, Prof. Dr. Klaus-Dieter, 18609 Ostseebad Binz (DE); Horn, Stefan, 17509 Loissin (DE)
(74) Vertreter: Schulz Junghans Patentanwälte PartGmbB

(56) Entgegenhaltungen:
- WO-A1-2014/040630
- JP-A- 2006 107 829
- KR-A- 20100 129 832
- US-A1- 2011 175 531
- US-A1- 2014 182 879
- KIM JAEHO ET AL: "Microwave-excited atmospheric-pressure plasma jets using a microstrip line", APPLIED PHYSICS LETTERS, A I P PUBLISHING LLC, US, Bd. 93, Nr. 19, 13. November 2008 (2008-11-13), Seiten 191505-191505, XP012112272, ISSN: 0003-6951, DOI: 10.1063/1.3025841
- Global Sources: "connectors supplier capability in mainland China" In: "connectors supplier capability in mainland China", 1 October 2004 (2004-10-01), XP055708185, ISBN: 978-962-785-371-8 page 71,

## Beschreibung

Die vorliegende Erfindung betrifft eine Plasmaerzeugungseinrichtung sowie ein Plasmaerzeugungssystem mit mehreren der erfindungsgemäßen Plasmaerzeugungseinrichtungen. Weiterhin betrifft die vorliegende Erfindung ein Verfahren zur Erzeugung von Plasma sowie ein Verfahren zur Desinfektion von Oberflächen, die nicht zum menschlichen oder tierischen Körper gehören, bzw. kein Gewebe des menschlichen oder tierischen Körper sind.

Es gibt unterschiedliche Ansätze, Plasmen im medizinischen Bereich zu nutzen. Neben der punktuellen Anwendung wird versucht, Plasmen auch flächig zu nutzen, wie zum Beispiel zur Behandlung von Wunden. Die bisher veröffentlichen Ansätze beziehen sich dabei überwiegend auf spezifische Anwendungsfälle und stellen keine Gesamt-Lösungen für unterschiedliche Anwendungszwecke dar. Übliche Plasmaerzeugungseinrichtungen sehen eine Superposition von Jet-Plasmen für die Abdeckung einer Fläche vor. Es werden dabei verschiedene Jet-Düsengeometrien genutzt.

Eine Alternative in der Anwendung von Plasmen besteht in der Verwendung von im Wesentlichen flächig ausgeführten Einrichtungen, mit denen eine dielektrisch behinderte Entladung (DBE) realisiert werden kann. Derartige Einrichtungen sind üblicherweise aus Festigkeitsgründen aus einem relativ festen bzw. starren Grundmaterial ausgeführt, weisen jedoch ggf. flexible Reaktionskammern auf. Eine Anpassung an bestimmte Topografien ist daher nur eingeschränkt möglich.

Insbesondere Plasmaerzeugungseinrichtungen, die zur Anwendung im medizinischen Bereich ausgestaltet sind, unterliegen relativ strengen Vorschriften hinsichtlich der Sicherheit gegenüber einer drohenden, aus der anliegenden Hochspannung resultierenden Verletzungsgefahr. Zudem sind derartige existierende Plasmaerzeugungseinrichtungen hinsichtlich ihrer kapazitiven Last nur technisch aufwendig skalierbar.

So offenbart die DE102007030915 A1 eine Vorrichtung zur Behandlung von Oberflächen mittels durch dielektrisch behinderte Entladung erzeugten Plasmas, wobei diese Vorrichtung eine flexible Wirk-Oberfläche aufweist, die unmittelbar an das Plasma angrenzt. Das Dielektrikum bildet dabei an der flexiblen Oberfläche Plasmen aus. Durch diese Flexibilität ist es möglich, auch eine unebene Fläche mit der Vorrichtung abzudecken und einer Plasmabehandlung zu unterziehen.

Aus der WO2013167693 A1 ist eine Vorrichtung zur Behandlung von Freiformflächen bekannt, die kaltes Atmosphärendruckplasma, welches in einer dielektrisch behinderten Oberflächenentladung entstanden ist, nutzt, um insbesondere Bereiche menschlicher oder tierischer Hautareale sowie pflanzlicher Oberflächen zu behandeln. Die dabei verwendete Hochspannungselektrode besteht aus einem flexiblen Draht oder einem elektrisch leitbaren Faden, dessen Isolationsschicht als Dielektrikum für die zu erzeugende dielektrisch behinderte Entladung dient. Diese Kombination von einem Draht bzw. Faden mit geringer Biegesteifigkeit und daran angeordneter dehnbarer Isolationsschicht ermöglicht es, die Hochspannungselektrode der Topografie der zu behandelnden Oberfläche anzupassen.

Aus der WO2011023478 A1 ist ebenfalls eine Vorrichtung zur Behandlung von Bereichen menschlicher oder tierischer Haut- bzw. Schleimhautoberflächen bekannt, welche zu diesem Zweck ein kaltes Atmosphärendruckplasma erzeugen kann. Auch die hier verwendete Elektrodenanordnung zur Erzeugung einer dielektrisch behinderten Oberflächenentladung lässt sich gekrümmten Oberflächen anpassen.

Aus der US 2014/0182879 A1 ist eine flexible flächige Elektrodenanordnung für eine dielektrisch behinderte Gasentladung bekannt, wobei eine Elektrode im Bereich einer Endfläche mit einem Kontaktierungselement kontaktiert und gegenüber der Umgebung elektrisch isoliert ist.

Aus der US 2011/0175531 A1 ist eine Plasmaerzeugniseinheit mit einem Resonatorring bekannt, wobei der Resonatorring mit einem Stecker verbunden wird, um eine Spannungsquelle anzuschließen, die den Resonatorring ährend des Betriebs mit Leistung versorgt. Bei bestimmten Ausführungsformen wird als Stecker ein Subminiatur-Koaxialstecker Typ A (SMA) verwendet.

Aus der JP 2006-107829 A ist ein Plasmagerät mit Mikrowellenanregung bekannt, die ebenfalls einen SMA Stecker umfasst.

Der vorliegenden Erfindung liegt die Aufgabe zugrunde, eine Plasmaerzeugungseinrichtung, ein Plasmaerzeugungssystem, ein Verfahren zur Erzeugung von Plasma sowie ein Verfahren zur Desinfektion von Oberflächen, die nicht zum menschlichen oder tierischen Körper gehören, bzw. kein Gewebe des menschlichen oder tierischen Körper sind, zur Verfügung zu stellen, mittels denen in einfacher, leicht handhabbarer, sicherer und effizienter Weise die Erzeugung von Plasma und dieses Plasma nutzend eine Desinfektion von Oberflächen möglich ist.

Diese Aufgabe wird durch die erfindungsgemäße Plasmaerzeugungseinrichtung gemäß Anspruch 1, durch das erfindungsgemäße Plasmaerzeugungssystem gemäß Anspruch 10, durch das erfindungsgemäße Verfahren zur Erzeugung von Plasma gemäß Anspruch 12 und durch das erfindungsgemäße Verfahren zur Desinfektion von Oberflächen, die nicht zum menschlichen oder tierischen Körper gehören,bzw. kein Gewebe des menschlichen oder tierischen Körper sind, gemäß Anspruch 14 gelöst. Vorteilhafte Ausgestaltungen der erfindungsgemäßen Plasmaerzeugungseinrichtung sind in den Unteransprüchen 2 bis 9 angegeben. Eine Ausgestaltung des erfindungsgemäßen Plasmaerzeugungssystems ist in Unteranspruch 11 angegeben und eine Ausgestaltung des Verfahrens zur Erzeugung von Plasma ist in Unteranspruch 13 angegeben.

Die erfindungsgemäße Plasmaerzeugungseinrichtung umfasst einen Elektrodenträger und eine erste Elektrode sowie eine zweite Elektrode, wobei die erste Elektrode am oder im Elektrodenträger angeordnet ist. Weiterhin umfasst die Plasmaerzeugungseinrichtung eine hochspannungsgeeignete Steckkontaktverbindung zur elektrischen Kontaktierung wenigstens einer der Elektroden, wobei die Steckkontaktverbindung einen Stecker sowie eine Aufnahmebuchse zur Aufnahme des Steckers aufweist und die Aufnahmebuchse fest am oder im Elektrodenträger angeordnet ist und mit einer der Elektroden elektrisch leitfähig verbunden ist. Die Plasmaerzeugungseinrichtung ist vorzugsweise als medizinisches Pflaster ausgestaltet. Die Elektroden sind dabei derart zueinander angeordnet, dass bei Anlage einer ausreichenden elektrischen Spannung an den Elektroden Plasma zwischen den Elektroden ausbildbar ist. Die Steckverbindung ist dabei für den Hochvolt-(HV)-Bereich ausgebildet, genauso wie die Elektroden, nämlich für eine Spannung von mindestens 1 kV. Vorzugsweise weist die Plasmaerzeugungseinrichtung die doppelte Spannungsfestigkeit (also mindestens 2 kV) auf. Entsprechend sind die Elektroden der Plasmaerzeugungseinrichtung ebenfalls für den genannten HV-Bereich ausgestaltet.

Gemäß der Erfindung ist die erste Elektrode fest an oder im Elektrodenträger angeordnet. Bei realisierter Steckverbindung ist mittels Kraft- und/oder Formschluss eine feste mechanische Verbindung zwischen dem Stecker und der Aufnahmebuchse hergestellt. Diese feste mechanische Verbindung ist vorzugsweise manuell herstellbar und manuell lösbar.

Die Plasmaerzeugungseinrichtung dient zur flächigen Behandlung von Oberflächen, insbesondere von Bereichen menschlicher oder tierischer Haut- bzw. Schleimhautoberflächen oder auch pflanzlichen Oberflächen bzw. organischen Gewebes. Mit ihr können demzufolge derartige Oberflächenbereiche desinfiziert werden. Dafür wird bevorzugt kaltes Atmosphärendruckplasma erzeugt. Vorteilhafterweise ist dabei die erste Elektrode als die geerdete Elektrode ausgeführt und die zweite Elektrode als die Hochspannungselektrode ausgeführt. Die erfindungsgemäße Plasmaerzeugungseinrichtung ist vorzugsweise für einen Wechselspannungsbereich von 10 kHz bis zu 1 MHz, insbesondere für einen Wechselspannungsbereich von 30 kHz bis 40 kHz ausgestaltet bzw. ausgelegt.

In der Plasmaerzeugungseinrichtung gemäß der Erfindung ist vorgesehen, dass der Stecker einen Steckerschaft und darin angeordnet eine Vertiefung aufweist, in welche ein Eingreifelement der Aufnahmebuchse kraft- und/oder formschlüssig eingreift bzw. eingreifen kann. Der Steckerschaft stellt damit den elektrischen Kontakt mit der Buchse her. Die dafür genutzte Vertiefung kann auch als sogenannte Hinterschneidung bezeichnet werden, in welche das Eingreifelement eingreift bzw. eingreifen kann. So gemäß der Erfindung weist der Stecker am Steckerschaft eine umlaufende Rille oder Nut auf, die die Vertiefung ausbildet, und die Aufnahmebuchse weist als Eingreifelement ein Federelement auf, welches beim Einschieben des Steckers in die Aufnahmebuchse vom Steckerschaft federnd nach außen gedrückt wird und bei Überlagerung der Vertiefung in diese Vertiefung eingreift. Die Aufnahmebuchse weist vorzugsweise die Form einer Schüssel auf, und der Stecker bzw. der Steckerschaft weist vorzugsweise die Form eines kraft- und/ oder formschlüssig in der Schüssel aufnehmbaren Noppens auf. Das Federelement kann dabei durch eine separate Feder an der Aufnahmebuchse ausgebildet sein oder aber auch durch einen zumindest teilweise umlaufenden Rand der Aufnahmebuchse, der am Schaft des Steckers anliegt und aufgrund elastischer Rückstellkraft in die am Schaft befindliche Vertiefung eingreifen kann.

Denkbar ist auch eine alternative Ausgestaltung, die nicht durch die beanspruchte Erfindung abgedeckt ist, in der der Stecker selbst ein Federelement aufweist und die Aufnahmebuchse eine Vertiefung aufweist, sodass bei realisierter Steckverbindung das Federelement des Steckers in die Vertiefung der Aufnahmebuchse einrastet und so die kraft- und/oder formschlüssige Verbindung herstellt.

In einer bevorzugten Ausgestaltung der erfindungsgemäßen Plasmaerzeugungseinrichtung ist vorgesehen, dass die Aufnahmebuchse mit der ersten Elektrode elektrisch leitfähig verbunden ist. Dadurch bildet die Aufnahmebuchse die Masseelektrode aus, die durch Anlage des Steckerschafts an der Aufnahmebuchse den Massekontakt zwischen Steckerschaft und erster Elektrode realisiert.

Weiterhin ist vorzugsweise vorgesehen, dass ein inneres Teil des Steckers mit der zweiten Elektrode elektrisch leitfähig verbindbar oder verbunden ist. Diese elektrisch leitfähige Verbindung kann zum Beispiel mittels eines weiteren Steckkontakts realisiert sein. Demzufolge bildet der Stecker vorteilhafterweise zwei Stromkontakte aus, nämlich außen über den Steckerschaft und die Anlage an der Aufnahmebuchse und innen über das innere Teil des Steckers. Dieses innere Teil ist zum Beispiel ein Stift, der elektrisch isoliert im Steckerschaft steckt, sodass der Steckerschaft die Aufnahmebuchse und damit die erste Elektrode elektrisch kontaktieren kann und das innere Teil des Steckers die zweite Elektrode elektrisch kontaktiert oder kontaktieren kann, sodass mittels des Steckers beide Stromkontakte zur Anlage der elektrischen Spannung an den Elektroden der Plasmaerzeugungseinrichtung realisiert werden. In einer alternativen Ausführungsform ist vorgesehen, dass die zweite Elektrode über einen extra Kontakt mit Spannung versorgt wird.

Der Stecker weist vorzugsweise einen Griffbereich auf, dessen Längsachse in Bezug zum Steckerschaft in einem Winkel von 60 ° bis 120 ° verläuft. Insbesondere bietet die Anordnung des Griffbereichs im Bezug zum Steckerschaft in einem Winkel von 90 ° eine gute Handhabbarkeit und den Vorteil eines relativ geringen benötigten Volumens im Bereich der Plasmaerzeugungseinrichtung.

Am Griffbereich kann eine Befestigungseinrichtung vorgesehen sein, mittels derer eine oder mehrere Stromleitungen elektrisch leitfähig mit dem Stecker verbindbar oder verbunden sind. Dadurch lässt sich in einfacher Weise von den elektrischen Stromleitungen über die Befestigungseinrichtung zum Steckerschaft und von diesem auf die Aufnahmebuchse sowie die Elektroden die zur Plasmaherstellung benötigte Spannung anlegen. Vorteilhafterweise ist die Befestigungseinrichtung ebenfalls als eine Steckverbindung ausgestaltet, welche kraft- und/oder formschlüssig die Stromleitungen an den Stecker anschließen.

In einer bevorzugten Ausgestaltungsform der Plasmaerzeugungseinrichtung ist deren Aufnahmebuchse als offenes Hohlniet im Elektrodenträger ausgeführt. Durch die Klemmung einer Elektrode beim Nieten des Hohlniets lässt sich in einfacher Weise eine elektrisch leitfähige Verbindung zwischen der Aufnahmebuchse und dieser Elektrode herstellen.

Weiterhin ist vorgesehen, dass zwischen der ersten Elektrode und der zweiten Elektrode ein Dielektrikum angeordnet ist, um mittels dielektrisch behinderter Entladung Plasma erzeugen zu können.

Es ist bevorzugt vorgesehen, dass die zweite Elektrode als Draht oder Kabel ausgeführt ist und das Dielektrikum die zweite Elektrode zumindest abschnittsweise und bevorzugt vollständig ummantelt.

Vorteilhafterweise ist das Dielektrikum ein Fluorpolymer. Insbesondere Fluorpolymere wie Polyvinylidenfluorid (PVDF) und Ethylen-Tetrafluorethylen (ETFE) wirken sich dabei hinsichtlich ihres Energiebedarfs sowie der Durchschlagsfestigkeit vorteilhaft aus. Alternativ kann als Dielektrikum auch ein Silikon eingesetzt werden.

In weiterer bevorzugter Ausgestaltung der Plasmaerzeugungseinrichtung ist vorgesehen, dass die zweite Elektrode mäanderförmig in einer Ebene verläuft, wobei die zweite Elektrode lineare Abschnitte aufweist, die mittels im Wesentlich nicht-linearer Abschnitte miteinander verbunden sind, sodass die alternierend angeordneten linearen Abschnitte und nicht-linearen Abschnitte die Mäanderform ausbilden. Es ist vorgesehen, dass die zweite Elektrode die erste Elektrode lediglich im Bereich von linearen Abschnitten des Mäanders überlagert. Derart wird erreicht, dass in Biege-Bereichen der Elektrode, die Vorzugs-Zündbereiche darstellen, Feldstärkeüberhöhungen vermieden werden können, so dass in diesen Bereichen die Gefahr der Ausbildung eines unkontrollierten Entladungskanals durch eine fehlende Gegenelektrode gemindert ist. Vorzugsweise sollten die linearen Abschnitte der zweiten Elektrode dabei parallel zueinander verlaufen, um eine gute Abdeckung der zu behandelnden Fläche mit einer ausreichenden Sicherheit gegen die Ausbildung eines im Fehlerfall gegebenenfalls auftretenden Entladungskanals zu kombinieren. Die Ebene, in der die zweite Elektrode mäanderförmig verläuft, muss dabei nicht unbedingt eine gerade Ebene sein, sondern sie kann auch eine gebogene Ebene sein bzw. von einer geraden Ebene in eine gebogene Ebene überführbar sein. Statt nicht-linearer bzw. gebogener Abschnitte der zweiten Elektrode kann auch eine Vielzahl von kurzen linearen Abschnitten angeordnet sein, deren Interpolation jedoch einen nicht-linearen Streckenverlauf ergibt.

In einer alternativen Ausführungsform ist vorgesehen, dass sich die zweite Elektrode spiralförmig in der Ebene erstreckt, wobei dabei die einzelnen Abschnitte der spiralförmigen zweiten Elektrode derart voneinander beabstandet sein sollten, dass kritische Feldstärkeüberhöhungen vermieden werden.

Weiterhin ist bevorzugt vorgesehen, dass die erste Elektrode ein elektrisch leitfähiges erstes textiles Gebilde ist. Dieses textile Gebilde kann zum Beispiel ein Gewebe, Gewirke, Gestrick, Geflecht, Nähgewirke, Vliesstoff oder Filz oder auch eine Kombination derartiger Gebilde sein, wobei auch die Ausbildung von räumlichen textilen Gebilden, sog. Körpergebilden, wie zum Beispiel textile Schläuche, nicht ausgeschlossen werden soll.

Weiterhin ist die erfindungsgemäße Plasmaerzeugungseinrichtung dann vorteilhaft ausgebildet, wenn der Elektrodenträger ein zweites textiles Gebilde ist und die erste Elektrode fest mit dem Elektrodenträger verbunden ist. In der bevorzugten Ausführungsform, in der sowohl der Elektrodenträger und die erste Elektrode textile Gebilde sind, sind sie vorzugsweise miteinander vernäht und/oder verklebt, sodass die Ebenen des Elektrodenträgers und der ersten Elektrode im Wesentlichen parallel zueinander verlaufen und beide zusammen flexibel plastisch und/oder elastisch biegbar sind, um sich zum Beispiel der Topografie einer bestimmten Körperpartie anpassen zu können.

Weiterhin kann auch die zweite Elektrode mit dem Elektrodenträger und/oder mit der ersten Elektrode vernäht sein. Dies ist insbesondere dann vorteilhaft, wenn die zweite Elektrode mäanderförmig verläuft. So kann zum Beispiel die zweite Elektrode ein mäanderförmig gebogenes Kabel oder ein Draht sein, welches bzw. welcher mit einem Dielektrikum isoliert ist. Die erste Elektrode ist dabei eine elektrisch leitfähige textile Schicht, wobei die zweite Elektrode mit der ersten Elektrode vernäht ist. Beide Elektroden sind mit dem Elektrodenträger in Form eines Grundtextils mechanisch verbunden, vorzugsweise ebenfalls mittels Vernähung.

Damit wird insgesamt eine flexible Einheit zur Verfügung gestellt, die auch als plasmaerzeugendes Pflaster bezeichnet werden kann. Diese dadurch hergestellt Einheit von erster Elektrode und Elektrodenträger hat vorzugsweise ein Elastizitätsmodul von max. 1*10⁹ N/m², insbesondere ein Elastizitätsmodul zwischen 0,1 *10⁹ N/m² und 0,5*10⁹ N/m².

In weiterer vorteilhafter Ausgestaltung der erfindungsgemäßen Plasmaerzeugungseinrichtung ist vorgesehen, dass diese eine Abstandseinrichtung aufweist, mit der die Einhaltung eines Abstands zwischen der zweiten Elektrode und einer mit der Plasmaerzeugungseinrichtung zu bedeckenden Oberfläche gewährleistet ist. Die Abstandseinrichtung ist dabei ein Distanzelement zum Beispiel in Form einer Schicht, die überwiegend an der Seite der zweiten Elektrode angeordnet ist, die der ersten Elektrode gegenüberliegt. Diese Schicht kann als ein Körper ausgeführt sein, der mehrere Aussparungen oder Schlitze aufweist, wobei diese Schlitze vorzugsweise parallel zu linearen Abschnitten des mäanderförmigen Verlaufs der zweiten Elektrode angeordnet sind. Dadurch kann in einfacher Weise erzeugtes Plasma sowie durch das Plasma veränderte Gase oder Gasgemische durch die Schlitze der abzudeckenden bzw. abgedeckten Oberfläche zugeführt werden. Die Abstandseinrichtung kann zum Beispiel aus einem relativ starren Hydrokolloidgel oder auch einem Polyurethanschaum bestehen. Sie kann dabei selbst noch von einer Deckschicht bedeckt sein, welche letztendlich bei Verwendung der erfindungsgemäßen Plasmaerzeugungseinrichtung als Wundpflaster auf dem Gewebe des zu behandelnden Körperteils zur Auflage kommt. Diese Deckschicht sollte als eine gaspermeable Membran ausgeführt sein, damit zwischen den Elektroden erzeugtes Plasma sowie durch das Plasma veränderte Gase oder Gasgemische durch die Deckschicht der zu behandelnden Oberfläche zugeleitet werden kann. Die Deckschicht kann dabei zum Beispiel als luftdurchlässiges Textil-Netzgewebe ausgeführt sein.

Das heißt, dass die erfindungsgemäße Plasmaerzeugungseinrichtung generell einen Schichtaufbau aufweisen kann, der ausgehend vom Elektrodenträger als Basiselement die folgenden Schichten aufweisen kann: Elektrodenträger, erste oder zweite Elektrode, die jeweils andere der ersten und zweiten Elektrode, Abstandseinrichtung. Gegebenenfalls kann die Abstandseinrichtung wie beschrieben mit einer Deckschicht versehen sein.

Die erfindungsgemäße Plasmaerzeugungseinrichtung kann somit als medizinische Manschette, Pflaster oder Wundabdeckung verwendet werden. Vorzugsweise umfasst sie eine Spannungsversorgungseinheit, mit der zur Einstellung der Intensität des Plasmas gepulst Spannung anlegbar ist.

Mit der erfindungsgemäßen Plasmaerzeugungseinrichtung lässt sich großflächig Plasma applizieren, da die erfindungsgemäße Plasmaerzeugungseinrichtung eine relativ große Fläche abdecken kann, wie zum Beispiel eine Fläche größer als 6 cm², vorzugsweise sogar größer als 10 cm². Aufgrund der vorzugsweisen verwendeten textilen Grundstoffe ist es flexibel unterschiedlichen Topografien anpassbar. Neben der Hochspannungselektrode, welche vorzugsweise aus einem elektrisch leitfähigen Draht mit einer Fluorpolymer- oder einer Silikon-Isolierung besteht, umfasst es noch eine Masseelektrode und ggf. eine Abstandseinrichtung. Die Hochspannungselektrode ist in günstiger Weise in einem charakteristischen Muster auf der Massenelektrode angeordnet. Aufgrund der Verwendung eines Fluorpolymers oder eines Silikons als Isolierung des Drahts lässt sich somit eine dielektrisch behinderte Entladung zur Herstellung des Plasmas realisieren.

Die Steckverbindung stellt eine sichere, hochspannungsfeste Verbindung zwischen einer Spannungsversorgungseinheit und den Elektroden dar. Die als Niet ausgestaltete Aufnahmebuchse dient dabei als Masseelektrode, wobei die Hochspannung am inneren Teil des Steckers anliegt, welches wiederum die zweite Elektrode, die vorzugsweise mäanderförmig vorliegt, kontaktiert.

Die Gestaltung der Plasmaerzeugungseinrichtung als Pflaster lässt eine einfache und kostengünstige Fertigung zu. Das Pflaster kann zur Einzelanwendung konzipiert sein, sodass eine jeweilige, üblicherweise aufwändige Desinfektion überflüssig ist. Aufgrund der relativ geringen Herstellungs- und Materialkosten lässt sich eine flächendeckende Versorgung medizinischer Einrichtungen gewährleisten.

Zur Lösung der Aufgabe wird weiterhin ein Plasmaerzeugungssystem zur Verfügung gestellt, welches mehrere erfindungsgemäße Plasmaerzeugungseinrichtungen umfasst, wobei die Plasmaerzeugungseinrichtungen im Wesentlichen flächendeckend angeordnet sind. Das bedeutet, dass die Gesamtheit der Plasmaerzeugungseinrichtungen, die das Plasmaerzeugungssystem umfasst, aus aneinander angrenzenden Plasmaerzeugungseinrichtungen besteht, die demzufolge eine relativ große geschlossene Fläche abdecken. Dabei können alle im System angeordneten Plasmaerzeugungseinrichtungen von derselben Spannungsquelle bzw. von derselben Versorgungseinheit mit Spannung versorgt werden. Die einzelnen Plasmaerzeugungseinrichtungen können in einfacher mechanischer Weise miteinander verbunden bzw. verbindbar sein, um den Verbund des Plasmaerzeugungssystems herzustellen. Aufgrund der geringen Biegesteifigkeit der einzelnen Plasmaerzeugungseinrichtungen und/oder der sie aneinander fixierenden mechanischen Verbindungen kann somit eine relativ große unebene Fläche im Wesentlichen lückenlos abgedeckt werden, wie zum Beispiel ein gewölbter Bereich eines menschlichen Oberschenkels. Die Kombination mehrerer als Pflaster ausgeführter Plasmaerzeugungseinrichtungen im erfindungsgemäßen Plasmaerzeugungssystem ermöglicht eine direkte plasmamedizinische Behandlung von relativ großen Arealen, wie sie zum Beispiel durch Brandwunden entstehen. Vorzugsweise sind die im Plasmaerzeugungssystem verwendeten Plasmaerzeugungseinrichtungen derart ausgestaltet, dass sie eine Maximalkapazität aufweisen. Mehrere der diese vorzugsweise gleiche Maximalkapazität aufweisenden Plasmaerzeugungseinrichtungen werden in einem Verbund angeordnet, sodass diese die gleiche Maximalkapazität aufweisenden einzelnen Plasmaerzeugungseinrichtungen im System energetisch optimal betreibbar sind.

Um die mit dem erzeugten Plasma erzeugte Wirkung einzustellen kann vorgesehen sein, dass die Plasmaerzeugungseinrichtungen des Plasmaerzeugungssystems selektiv ansteuerbar sind. Somit lassen sich an unterschiedlichen Plasmaerzeugungseinrichtungen unterschiedliche Spannungen anlegen, wodurch sich wiederum unterschiedliche Feldstärken des Plasmas an unterschiedlichen Positionen des Plasmaerzeugungssystems erzeugen lassen. Dies hat den Vorteil der zielgerichteten, partiell möglichen Plasmabehandlung mit einem einzigen Plasmaerzeugungssystem.

Zu diesem Zweck kann das Plasmaerzeugungssystem weiterhin eine Steuerungseinrichtung aufweisen, mit der die Spannung selektiv an den einzelnen Plasmaerzeugungseinrichtungen einstellbar ist. Die Summe der einzelnen Kapazitäten der Plasmaerzeugungseinrichtungen sollte dabei nicht die von der Steuerungseinrichtung einstellbaren oder umsetzbaren Kapazität überschreiten. Das erfindungsgemäße Plasmaerzeugungssystem ist dabei nicht auf eine bestimmte Anzahl der integrierten Plasmaerzeugungseinrichtungen und auch nicht auf bestimmte Größen der Plasmaerzeugungseinrichtungen eingeschränkt, sondern kann beliebig skaliert werden.

Zur Lösung der Aufgabe wird weiterhin ein Verfahren zur Erzeugung von Plasma zur Verfügung gestellt, welches insbesondere zur Erzeugung kalten Atmosphärendruckplasmas geeignet ist. Das Plasma sowie durch das Plasma veränderte Gase oder Gasgemische werden mittels einer dielektrisch behinderten Entladung erzeugt, wobei eine erfindungsgemäße Plasmaerzeugungseinrichtung oder auch ein erfindungsgemäßes Plasmaerzeugungssystem genutzt wird, an deren jeweilige ersten und zweiten Elektroden die elektrische Hochspannung angelegt wird. Vorzugsweise sollte die Hochspannung einen Frequenzbereich von 10 kHz bis zu 1 MHz aufweisen.

Das Verfahren zur Erzeugung von Plasma kann dabei mit einem erfindungsgemäßen Plasmaerzeugungssystem derart durchgeführt werden, dass an einzelnen und gegebenenfalls an allen Plasmaerzeugungseinrichtungen des Plasmaerzeugungssystems die angelegte Spannung separat eingestellt wird, um partiell unterschiedliche Plasmabehandlungen zu ermöglichen.

Ein weiterer Aspekt der vorliegenden Erfindung ist ein Verfahren zur Desinfektion von Oberflächen, die nicht zum menschlichen oder tierischen Körper gehören, bzw. kein Gewebe des menschlichen oder tierischen Körper sind, bei dem das erfindungsgemäße Verfahren zur Erzeugung von Plasma in einem solchen Abstand zur zu desinfizierenden Oberfläche durchgeführt wird, dass zumindest ein Teilbereich der zu desinfizierenden Oberfläche mit erzeugtem Plasma sowie mit durch das Plasma veränderten Gase oder Gasgemischen in Kontakt kommt. Dabei ist dieses Verfahren nicht nur zur Behandlung von pflanzlichen Oberflächen ausgestaltet, sondern kann auch für alle anorganischen Oberflächen, wie zum Beispiel Werkzeug-Oberflächen, genutzt werden.

Die Erfindung betrifft ebenfalls die Verwendung der erfindungsgemäßen Plasmaerzeugungseinrichtung bzw. des erfindungsgemäßen Plasmaerzeugungssystems zur Desinfektion von Oberflächen, die nicht zum menschlichen oder tierischen Körper gehören, bzw. kein Gewebe des menschlichen oder tierischen Körper sind, wie anorganischen Derart löst die vorliegende Erfindung die Gesamtproblematik der Behandlung von nicht-planaren Oberflächen, bevorzugt von inhomogenen biologischen Oberflächen, die nicht zum menschlichen oder tierischen Körper gehören, bzw. kein Gewebe des menschlichen oder tierischen Körper sind, wobei die Behandlung in einfacher, sicherer und kostengünstiger Weise erfolgen kann.

Die Erfindung wird im Folgenden anhand der in den beiliegenden Zeichnungen dargestellten Ausführungsbeispiele erläutert.

Es zeigt
- Fig. 1:: eine erfindungsgemäße Plasmaerzeugungseinrichtung in Draufsicht,
- Fig. 2:: eine erfindungsgemäße Plasmaerzeugungseinrichtung in Ansicht von der Seite,
- Fig. 3:: ein erfindungsgemäßes Plasmaerzeugungssystem,
- Fig. 4:: unterschiedliche Varianten des Schicht-Aufbaus der erfindungsgemäßen Plasmaerzeugungseinrichtung in den Varianten a), b) und c),
- Fig. 5:: eine weitere, perspektivische Darstellung eines erfindungsgemäßen Plasmaerzeugungssystems.

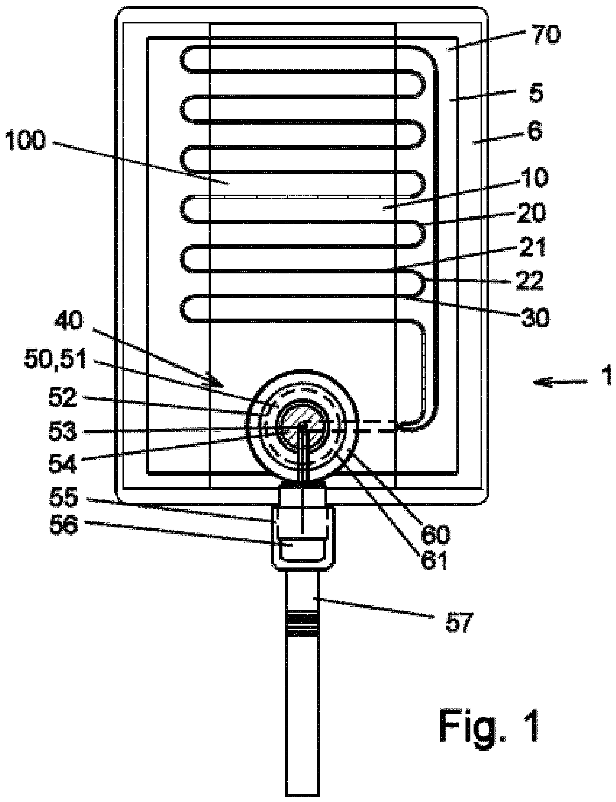

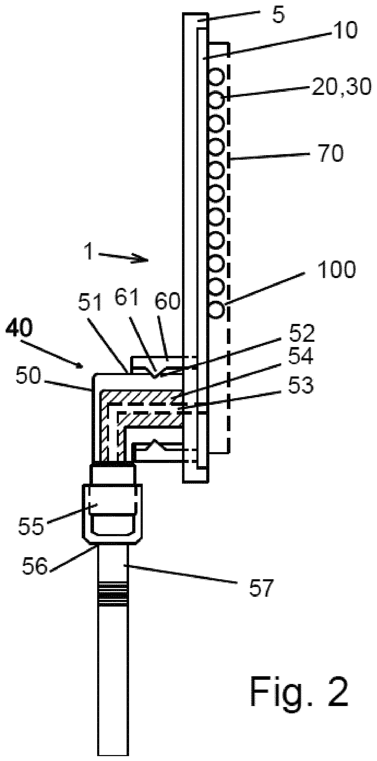

Die erfindungsgemäße Plasmaerzeugungseinrichtung 1 umfasst, wie in den Fig. 1 und 2 ersichtlich ist, einen Elektrodenträger 5, der im Wesentlichen als ein flächiges Material ausgeführt ist. Vorzugsweise ist dieser Elektrodenträger ein textiles Gebilde. Auf dem Elektrodenträger 5 ist eine erste Elektrode 10 angeordnet, welche vorzugsweise ebenfalls ein textiles Gebilde ist und hier in einer Streifenform vorliegt. Diese erste Elektrode 10 wird gebietsweise von einer zweiten Elektrode 20 überlagert, welche mäanderförmig parallel zur Ebene der ersten Elektrode 10 sowie des Elektrodenträgers 5 angeordnet ist. Die Mäander-Form der zweiten Elektrode 20 umfasst lineare Abschnitte 21, die im Wesentlichen parallel zueinander angeordnet sind, und die durch nicht-lineare Abschnitte 22 bzw. Krümmungen miteinander verbunden sind. Die zweite Elektrode 20 ist von einer Isolationsschicht umhüllt, die als Dielektrikum 30 ausgeführt ist.

Die Plasmaerzeugungseinrichtung 1 umfasst weiterhin eine Steckkontaktverbindung 40, die wiederum einen Stecker 50 und eine Aufnahmebuchse 60 aufweist. Die Aufnahmebuchse 60 ist fest mit dem Elektrodenträger 5 und in der hier dargestellten Ausführungsvariante auch mit der ersten Elektrode 10 verbunden. Die Aufnahmebuchse 60 ist hierbei als ein Hohlniet ausgestaltet, in welches ein Stecker 50 der Steckkontaktverbindung 40 eingesteckt ist. Die äußere Oberfläche des Steckerschafts 51 liegt dabei mit seiner Außenseite an der Innenseite der ringförmig ausgestalteten Aufnahmebuchse 60 an. Außerhalb des eingesteckten Steckerschafts 51 kann dieser selbstverständlich mit einem Isolator bedeckt sein.

Wie insbesondere aus Fig. 2 ersichtlich ist, ist am Steckerschaft 51 eine Vertiefung 52 angeordnet, in welche im eingesteckten Zustand des Steckers 50 ein Eingreifelement 61 der Aufnahmebuchse 60 kraft- und/oder formschlüssig eingreift, um den Stecker 50 in der Aufnahmebuchse 60 zu fixieren. Dadurch wird ebenfalls der elektrische Kontakt zwischen dem Steckerschaft 51 und der Aufnahmebuchse 60 und demzufolge auch mit der ersten Elektrode 10 realisiert.

Insbesondere aus Fig. 2 ist ersichtlich, dass sich an den Steckerschaft 51 in einem Winkel von ca. 90 Grad ein Griffbereich 55 anschließt, an welchen mittels einer Befestigungseinrichtung 56 ein Kabel angeschlossen ist.

Zur Erläuterung des inneren Aufbaus des Steckers 50 ist wiederum auf Fig. 1 zu erweisen, in der aus Gründen der Übersichtlichkeit der Bereich des Griffbereichs 55, welcher den Steckerschaft 51 überlagert, nicht dargestellt ist, um das innere Teil 53 des Steckers 50 sichtbar zu machen Es ist ersichtlich, dass ein zentral im Griffbereich 55 verlaufender elektrischer Kontakt ein ebenfalls zentral im Steckerschaft 51 und von diesem durch eine Isolation 54 elektrisch isoliert angeordnetes inneres Teil 53 kontaktiert. Dieses innere Teil 53 ist mit der zweiten Elektrode 20 elektrisch leitfähig verbunden oder auch in einfacher Weise elektrisch leitfähig verbindbar, wie zum Beispiel mittels einer weiteren Steckverbindung. Die zweite Elektrode 20 ist bevorzugt die Hochspannungselektrode, sodass die Hochspannung an diese zweite Elektrode 20 über das innere Teil 53 des Steckers 50 angelegt wird, wobei die erste Elektrode 10 hier als Masseelektrode dient.

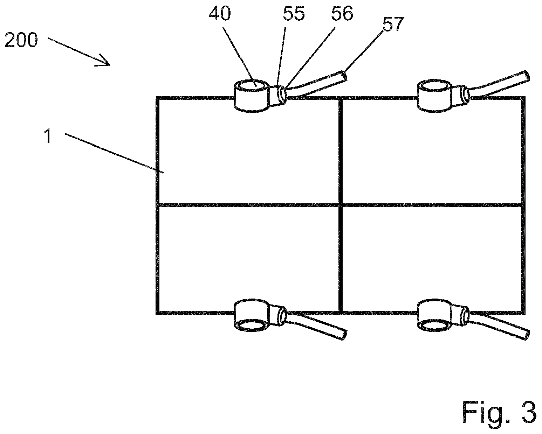

Bei Anlage der Spannung an die Elektroden 10, 20 und die Verwendung des Dielektrikums als Ummantelung der zweiten Elektrode 20 wird somit Plasma in einer dielektrisch behinderten Entladung erzeugt. Um Vorzugs-Zündbereichen aufgrund von Feldstärkeüberhöhungen vorzubeugen, überlagert die mäanderförmig verlaufende zweite Elektrode 20 die streifenförmig ausgebildete erste Elektrode 10 nur in dem Bereich der linearen Abschnitte 21 der zweiten Elektrode 20. Demzufolge erfolgt eine Plasma-Ausbildung nur zwischen den linearen Abschnitten 21 der zweiten Elektrode 20 und der ersten Elektrode 10 und nicht im Bereich der nicht-linearen Abschnitte 22 bzw. Krümmungen zwischen den linearen Abschnitten 21. Aufgrund dessen, dass als Elektrodenträger 5 sowie auch als erste Elektrode 10 und zweite Elektrode 20 flexible Materialien mit geringer Biegesteifigkeit verwendet werden, lässt sich die erfindungsgemäße Plasmaerzeugungseinrichtung 1 um sämtliche drei Koordinatenachsen biegen und demzufolge auch gewölbten Oberflächen anpassen. Durch eine Modularisierung lässt sich somit das in Fig. 3 dargestellte Plasmaerzeugungssystem realisieren, welches mehrere, hier vier dargestellte, einzelne Plasmaerzeugungseinrichtungen zu einem System zusammenfasst, wobei die Plasmaerzeugungseinrichtungen 1 derart angeordnet sind, dass sie zusammen ein rohr- bzw. tubusförmiges Gebilde realisieren, um damit zum Beispiel einen Abschnitt eines Unterarms vollständig abzudecken. Das hierdurch hergestellte Plasmaerzeugungssystem 200 kann nahezu beliebig skaliert werden, wobei die an die Steckkontaktverbindungen 40 angeschlossenen Kabel vorzugsweise alle an dieselbe, hier nicht darstellte, Spannungsversorgungseinheit angeschlossen sind und demzufolge alle einzelnen Plasmaerzeugungseinrichtungen 1 zusammen angesteuert und betrieben werden können.

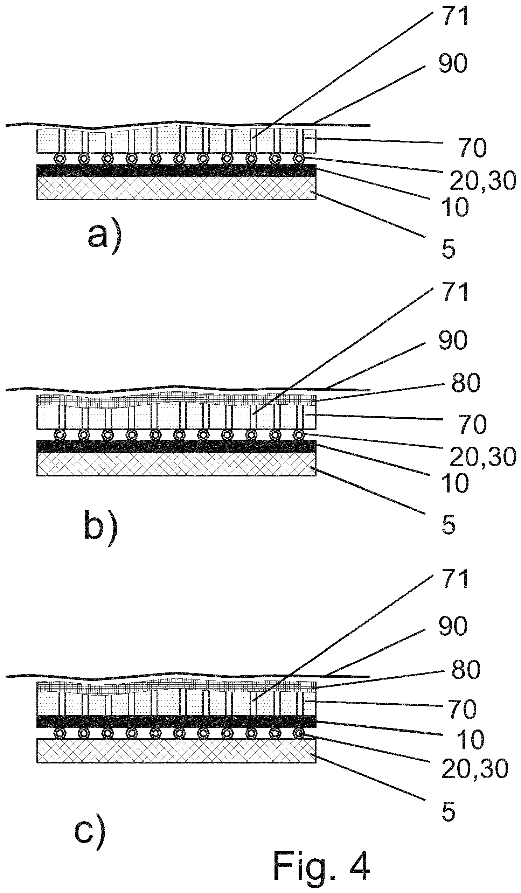

Mögliche Varianten des Schicht-Aufbaus der erfindungsgemäßen Plasmaerzeugungseinrichtung sind aus den drei Varianten a), b) und c) der Fig. 4 ersichtlich. Allen Varianten ist dabei als Basiselement der Elektrodenträger 5 gemeinsam. Auf diesem ist in den Varianten a) und b) die erste Elektrode 10 angeordnet, auf der wiederum die zweite Elektrode 20, zum Beispiel in der beschriebenen Mäanderform, mit dem umhüllenden Dielektrikum 30 angeordnet ist. Vorzugsweise sind der Elektrodenträger und die erste Elektrode 10 miteinander verklebt und/oder vernäht. Ebenso bietet es sich an, die zweite Elektrode 20 auch mit dem Elektrodenträger 5 bzw. der ersten Elektrode 10 zu vernähen bzw. zu verkleben.

Die zweite Elektrode 20 sowie das umhüllende Dielektrikum 30 sind von einer Abstandseinrichtung 70 überdeckt, die im Wesentlichen eine relativ weiche Schicht ausbildet und Aussparungen 71 aufweist, durch die zwischen den Elektroden 10, 20 erzeugtes Plasma in Richtung der behandelnden Oberfläche 90 hindurchtreten kann. In Variante a) liegt die Abstandseinrichtung 70 direkt an der zu behandelnden Oberfläche 90 an. In Variante b) ist die Abstandseinrichtung 70 noch mit einer Deckschicht 80 versehen, die gaspermeabel ausgebildet ist, sodass zwischen den Elektroden 10, 20 erzeugtes Plasma durch die Aussparungen 71 der Abstandseinrichtung 70 hindurchtreten kann und derart durch die gaspermeable Deckschicht 80 an die zu behandelnde Oberfläche 90 gelangen kann.

Variante c) in Fig. 4 weist einen abweichenden Aufbau auf, bei dem auf dem Elektrodenträger 5 die zweite Elektrode 20 mit dem umhüllenden Dielektrikum 30 angeordnet ist, und diese zweite Elektrode 20 wiederum von der ersten Elektrode 10 abgedeckt ist. Dadurch, dass die ersten Elektrode 10 vorzugsweise als textiles Gebilde ausgeführt ist, kann zwischen den Elektroden 10, 20 entstehendes Plasma durch die erste Elektrode 10 in Richtung der zu behandelnden Oberfläche 90 geführt werden, wobei es durch die Aussparungen 71 der abdeckenden Abstandseinrichtung 70 geführt wird und durch die darüber angeordnete Deckschicht 80 hindurchtritt. Dabei ist die Ausführungsform gem. c) in Fig. 4 nicht auf die Anordnung der Deckschicht 80 eingeschränkt, sondern sie kann entsprechend wie Ausführung a) auch ohne Deckschicht 80 ausgeführt sein.

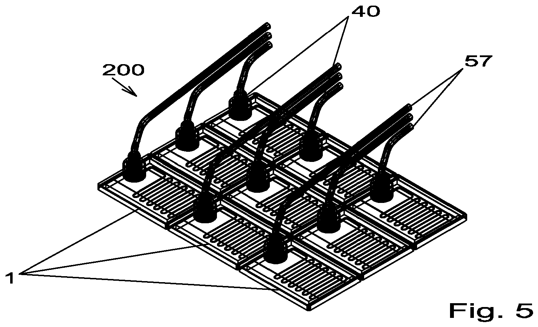

In Fig. 5 ist eine weitere, perspektivische Darstellung eines erfindungsgemäßen Plasmaerzeugungssystems 200 dargestellt. Es ist ersichtlich, dass das Plasmaerzeugungssystem auch in dieser Ausführungsform mehrere einzelne Plasmaerzeugungseinrichtungen 1 aufweist, die mechanisch miteinander verbunden sind. Jede der Plasmaerzeugungseinrichtungen 1 weist die beschriebene Steckkontaktverbindung 40 auf, die mittels eines Kabels 57 an eine hier nicht dargestellte Steuerungseinrichtung aufweisen, mit der die Spannung selektiv an den einzelnen Plasmaerzeugungseinrichtungen einstellbar ist. Die Summe der einzelnen Kapazitäten der Plasmaerzeugungseinrichtungen 1 überschreitet dabei nicht die von der Steuerungseinrichtung einstellbare oder umsetzbare Kapazität. Es ist ersichtlich, dass durch die selektive Ansteuerbarkeit bzw. Einstellbarkeit der an einer Plasmaerzeugungseinrichtung 1 anliegenden Spannung die von einer jeweiligen Plasmaerzeugungseinrichtung erzeugte Plasma-Intensität im Wesentlichen ortsunabhängig gesteuert werden kann.

### Bezugszeichenliste

- Plasmaerzeugungseinrichtung: 1
- Elektrodenträger: 5
- erste Elektrode: 10
- zweite Elektrode: 20
- linearer Abschnitt: 21
- nicht-linearer Abschnitt: 22
- Dielektrikum: 30
- Steckkontaktverbindung: 40
- Stecker: 50
- Steckerschaft: 51
- Vertiefung: 52
- inneres Teil: 53
- Isolation: 54
- Griffbereich: 55
- Befestigungseinrichtung: 56
- Kabel: 57
- Aufnahmebuchse: 60
- Eingreifelement: 61
- Abstandseinrichtung: 70
- Aussparung: 71
- Deckschicht: 80
- Zu behandelnde Oberfläche: 90
- Plasma: 100
- Plasmaerzeugungssystem: 200

## Patentansprüche

1. Plasmaerzeugungseinrichtung (1), umfassend einen Elektrodenträger (5) und eine erste Elektrode (10) sowie eine zweite Elektrode (20), wobei die erste Elektrode (10) am oder im Elektrodenträger (5) angeordnet ist, und weiterhin umfassend eine hochspannungsgeeignete Steckkontaktverbindung (40) zur elektrischen Kontaktierung wenigstens einer der Elektroden (10,20), wobei die Steckkontaktverbindung (40) einen Stecker (50) sowie eine Aufnahmebuchse (60) zur Aufnahme des Steckers (50) aufweist und die Aufnahmebuchse (60) fest am oder im Elektrodenträger (5) angeordnet ist und mit einer der Elektroden (10,20) elektrisch leitfähig verbunden ist, wobei der Stecker (50) einen Steckerschaft (51) und darin angeordnet eine Vertiefung (52) aufweist, in welche ein Eingreifelement (61) der Aufnahmebuchse (60) kraft- und/ oder formschlüssig eingreift, wobei der Stecker (50) am Steckerschaft (51) eine umlaufende Rille oder Nut aufweist, die die Vertiefung (52) ausbildet,
**dadurch gekennzeichnet, dass**
die Aufnahmebuchse (60) als Eingreifelemen (61) ein Federelement aufweist, welches beim Einschieben des Steckers (50) in die Aufnahmebuchse (60) vom Steckerschaft (51) federnd nach außen gedrückt wird und bei Überlagerung der Vertiefung in diese Vertiefung eingreift.

2. Plasmaerzeugungseinrichtung nach Anspruch 1, wobei die Aufnahmebuchse (60) mit der ersten Elektrode (10) elektrisch leitfähig verbunden ist.

3. Plasmaerzeugungseinrichtung nach Anspruch 2, wobei ein inneres Teil (53) des Steckers (50) mit der zweiten Elektrode (20) elektrisch leitfähig verbunden oder verbindbar ist.

4. Plasmaerzeugungseinrichtung nach einem der vorhergehenden Ansprüche, wobei die Aufnahmebuchse (60) als offenes Hohlniet im Elektrodenträger (5) ausgeführt ist.

5. Plasmaerzeugungseinrichtung nach einem der vorhergehenden Ansprüche, wobei zwischen der ersten Elektrode (10) und der zweiten Elektrode (20) ein Dielektrikum (30) angeordnet ist.

6. Plasmaerzeugungseinrichtung nach Anspruch 5, wobei dass die zweite Elektrode (20) als Draht oder Kabel ausgeführt ist und das Dielektrikum (30) die zweite Elektrode (20) zumindest abschnittsweise ummantelt.

7. Plasmaerzeugungseinrichtung nach einem der vorhergehenden Ansprüche, wobei die zweite Elektrode (20) mäanderförmig in einer Ebene verläuft, wobei die zweite Elektrode (20) lineare Abschnitte (21) aufweist, die mittels im Wesentlichen nicht-linearer Abschnitte (22) miteinander verbunden sind, so dass die alternierend angeordneten linearen Abschnitte (21) und nicht-linearen Abschnitte (22) die Mäanderform ausbilden, und die zweite Elektrode (20) die erste Elektrode (10) lediglich im Bereich von linearen Abschnitten (22) des Mäanders überlagert.

8. Plasmaerzeugungseinrichtung nach einem der vorhergehenden Ansprüche, wobei die erste Elektrode (10) ein elektrisch leitfähiges erstes textiles Gebilde ist.

9. Plasmaerzeugungseinrichtung nach einem der vorhergehenden Ansprüche, wobei die Plasmaerzeugungseinrichtung (1) eine Abstandseinrichtung (70) aufweist, mit der die Einhaltung eines Abstandes zwischen der zweiten Elektrode (20) und einer mit der Plasmaerzeugungseinrichtung (1) zu bedeckenden Oberfläche (90) gewährleistet ist.

10. Plasmaerzeugungssystem (200), umfassend mehrere Plasmaerzeugungseinrichtungen (1) gemäß einem der Ansprüche 1 bis 9, wobei die Plasmaerzeugungseinrichtungen (1) im Wesentlichen flächendeckend angeordnet sind.

11. Plasmaerzeugungssystem (200) nach Anspruch 10, wobei die Plasmaerzeugungseinrichtungen (1) selektiv ansteuerbar sind.

12. Verfahren zur Erzeugung von Plasma, mittels einer dielektrisch behinderten Entladung, bei dem eine Plasmaerzeugungseinrichtung (1) gemäß einem der Ansprüche 1 bis 9 oder ein Plasmaerzeugungssystem (200) gemäß einem der Ansprüche 10 und 11 bereit gestellt wird und elektrische Hochspannung an die jeweilige erste Elektrode (10) und zweite Elektrode (20) angelegt wird.

13. Verfahren zur Erzeugung von Plasma nach Anspruch 12, wobei ein Plasmaerzeugungssystem (200) gemäß einem der Ansprüche 10 und 11 bereit gestellt wird und an einzelnen Plasmaerzeugungseinrichtungen (1) des Plasmaerzeugungssystems (200) die angelegte Spannung separat eingestellt wird.

14. Verfahren zur Desinfektion von Oberflächen, die nicht zur menschlichen oder tierischen Körper gehören, bzw. kein Gewebe des menschlichen oder tierischen Körper sind, bei dem das Verfahren zur Erzeugung von Plasma gemäß einem der Ansprüche 12 und 13 in einem solchen Abstand zur zu desinfizierenden Oberfläche (90) durchgeführt wird, dass zumindest ein Teilbereich der zu desinfizierenden Oberfläche (90) mit erzeugtem Plasma sowie mit durch das Plasma veränderten Gase oder Gasgemischen in Kontakt kommt.

## Claims

1. A plasma generating device (1), comprising an electrode carrier (5) and a first electrode (10) as well as a second electrode (20), wherein the first electrode (10) is arranged on or in the electrode carrier (5), and further comprising a high-voltage suitable plug contact connection (40) for electrically contacting at least one of the electrodes (10,20), wherein the plug contact connection (40) has a plug (50) as well as a receiving port (60) for receiving the plug (50) and the receiving port (60) is securely arranged on or in the electrode carrier (5) and is electroconductively connected to one of the electrodes (10,20), wherein the plug (50) has a plug shaft (51) and a recess (52) arranged therein, in which recess an engaging element (61) of the receiving port (60) is force- and/or form-fittingly engaged, wherein the plug (50) has a circumferential channel or groove on the plug shaft (51) which forms the recess (52),
**characterized in that** the receiving port (60) has a spring element as an engaging element (61) which is resiliently pressed outward by the plug shaft (51) during insertion of the plug (50) into the receiving port (60) and which engages with the recess when overlapping with said recess.

2. The plasma generating device according to Claim 1, wherein the receiving port (60) is electroconductively connected to the first electrode (10).

3. The plasma generating device according to Claim 2, wherein an inner part (53) of the plug (50) is electroconductively connected or connectable to the second electrode (20).

4. The plasma generating device according to any one of the preceding claims, wherein the receiving port (60) is configured as an open hollow rivet in the electrode carrier (5).

5. The plasma generating device according to any one of the preceding claims, wherein a dielectric (30) is arranged between the first electrode (10) and the second electrode (20).

6. The plasma generating device according to Claim 5, wherein the second electrode (20) is configured as a wire or a cable and the dielectric (30) wraps the second electrode (20) at least in sections.

7. The plasma generating device according to any one of the preceding claims, wherein the second electrode (20) runs meanderingly in one plane, wherein the second electrode (20) has linear sections (21) which are connected to each other by substantially non-linear sections (22) such that the alternatingly arranged linear sections (21) and non-linear sections (22) form the meandering form, and the second electrode (20) only overlaps the first electrode (10) in the region of linear sections (22) of the meandering.

8. The plasma generating device according to any one of the preceding claims, wherein the first electrode (10) is an electroconductive first textile construction.

9. The plasma generating device according to any one of the preceding claims, wherein the plasma generating device (1) has a spacing device (70) with which a spacing between the second electrode (20) and a surface (90) which is to be covered by the plasma generating device (1) is maintained.

10. A plasma generating system (200), comprising several plasma generating devices (1) according to any one of Claims 1 to 9, wherein the plasma generating devices (1) are substantially arranged over the entire surface.

11. The plasma generating system (200) according to Claim 10, wherein the plasma generating devices (1) are selectively controllable.

12. A method for generating plasma by means of a dielectric barrier discharge for which a plasma generating device (1) according to any one of Claims 1 to 9 or a plasma generating system (200) according to any one of Claims 10 and 11 is provided and electrical high-voltage is applied to the first electrode (10) and the second electrode (20) respectively.

13. The method for generating plasma according to Claim 12, wherein a plasma generating system (200) according to any one of Claims 10 and 11 is provided and the applied voltage is adjusted separately for individual plasma generating devices (1) of the plasma generating system (200).

14. A method for disinfecting surfaces which do not belong to human or animal bodies or which are not human or animal body tissue for which the method for generating plasma according to any one Claims 12 and 13 is carried out at such a distance from the surface (90) to be disinfected that at least a partial region of the surface (90) to be disinfected comes into contact with generated plasma as well as with gases or gas mixtures altered by the plasma.

## Revendications

1. Dispositif de génération de plasma (1), comprenant un support d'électrodes (5) et une première électrode (10) ainsi qu'une seconde électrode (20), dans lequel la première électrode (10) est disposée sur ou dans le support d'électrodes (5), et comprenant en outre une connexion par contact à fiche (40) convenant à la haute tension pour la mise en contact électrique d'au moins une des électrodes (10, 20), dans lequel la connexion par contact à fiche (40) présente un connecteur (50) ainsi qu'une douille de réception (60) pour la réception du connecteur (50), et la douille de réception (60) est disposée de manière fixe sur ou dans le support d'électrodes (5) et est connectée de manière électriquement conductrice à une des électrodes (10, 20), dans lequel le connecteur (50) présente une tige de connecteur (51) et un renfoncement (52) disposé en son sein dans lequel un élément d'engagement (61) de la douille de réception (60) s'engage par adhérence et/ou conjugaison de formes, dans lequel le connecteur (50) présente sur la tige de connecteur (51) une cannelure ou rainure périphérique qui forme le renfoncement (52), **caractérisé en ce que** la douille de réception (60) présente en tant qu'élément d'engagement (61) un élément élastique qui est poussé de manière élastique vers l'extérieur par la tige de connecteur (51) lors de l'insertion du connecteur (50) dans la douille de réception (60) et s'engage dans le renfoncement lors de la superposition de ce renfoncement.

2. Dispositif de génération de plasma selon la revendication 1, dans lequel la douille de réception (60) est connectée de manière électriquement conductrice à la première électrode (10).

3. Dispositif de génération de plasma selon la revendication 2, dans lequel une partie interne (53) du connecteur (50) est connectée ou peut être connectée de manière électriquement conductrice à la seconde électrode (20).

4. Dispositif de génération de plasma selon une des revendications précédentes, dans lequel la douille de réception (60) est réalisée en tant que rivet creux ouvert dans le support d'électrodes (5).

5. Dispositif de génération de plasma selon une des revendications précédentes, dans lequel un diélectrique (30) est disposé entre la première électrode (10) et la seconde électrode (20).

6. Dispositif de génération de plasma selon la revendication 5, dans lequel la seconde électrode (20) est réalisée en tant que fil ou câble et le diélectrique (30) enveloppe la seconde électrode (20) au moins par sections.

7. Dispositif de génération de plasma selon une des revendications précédentes, dans lequel la seconde électrode (20) s'étend en forme de méandre dans un plan, dans lequel la seconde électrode (20) présente des sections linéaires (21) qui sont connectées les unes aux autres au moyen de sections essentiellement non linéaires (22) de sorte que les sections linéaires (21) et sections non linéaires (22) disposées en alternance forment la forme de méandre, et la seconde électrode (20) se superpose à la première électrode (10) uniquement dans la région de sections linéaires (22) du méandre.

8. Dispositif de génération de plasma selon une des revendications précédentes, dans lequel la première électrode (10) est un premier produit textile électriquement conducteur.

9. Dispositif de génération de plasma selon une des revendications précédentes, dans lequel le dispositif de génération de plasma (1) présente un écarteur (70) avec lequel le respect d'un écart entre la seconde électrode (20) et une surface (90) à recouvrir du dispositif de génération de plasma (1) est garanti.

10. Système de génération de plasma (200), comprenant plusieurs dispositifs de génération de plasma (1) selon une des revendications 1 à 9, dans lequel les dispositifs de génération de plasma (1) sont disposés de manière essentiellement couvrante.

11. Système de génération de plasma (200) selon la revendication 10, dans lequel les dispositifs de génération de plasma (1) peuvent être commandés de manière sélective.

12. Procédé de génération de plasma, au moyen d'une décharge à barrière diélectrique, lors duquel un dispositif de génération de plasma (1) selon une des revendications 1 à 9 ou un système de génération de plasma (200) selon une des revendications 10 et 11 est mis à disposition et une haute tension électrique est appliquée aux première électrode (10) et seconde électrode (20) respectives.

13. Procédé de génération de plasma selon la revendication 12, dans lequel un système de génération de plasma (200) selon une des revendications 10 et 11 est mis à disposition et la tension appliquée est réglée séparément sur des dispositifs de génération de plasma individuels (1) du système de génération de plasma (200).

14. Procédé de désinfection de surfaces qui n'appartiennent pas aux corps humains ou animaux, ou ne sont pas un tissu du corps humain ou animal, lors duquel le procédé de génération de plasma selon une des revendications 12 et 13 est mis en œuvre à un écart de la surface à désinfecter (90) tel qu'au moins une région partielle de la surface à désinfecter (90) vient en contact avec du plasma généré ainsi qu'avec des gaz ou mélanges gazeux modifiés par le plasma.
